# EUROPEAN PATENT SPECIFICATION

(11) **EP 3 845 149 B1**
(45) Date of publication and mention of the grant of the patent: **11.06.2025**
(21) Application number: 19867273.5
(22) Date of filing: 26.09.2019
(51) Int. Cl.: A61B 17/115

(54) **ADHESION PROMOTION DEVICE**
HAFTVERMITTLUNGSVORRICHTUNG
DISPOSITIF FAVORISANT L'ADHÉRENCE

(30) Priority: 27.09.2018 JP 2018182245
(43) Date of publication of application: 07.07.2021
(73) Proprietor: TERUMO Kabushiki Kaisha, Tokyo 151-0072 (JP)
(72) Inventor: ARAMAKI, Naoki, Ashigarakami-gun, Kanagawa 259-0151 (JP); KAI, Miho, Ashigarakami-gun, Kanagawa 259-0151 (JP); HATA, Mayu, Ashigarakami-gun, Kanagawa 259-0151 (JP)
(74) Representative: Casalonga
(86) International application number: PCT/JP2019/038027
(87) International publication number: WO 2020/067372

(56) References cited:
- EP-A1- 1 647 231
- WO-A1-2018/025212
- WO-A1-2018/025212
- JP-A- 2005 529 675
- JP-A- 2006 255 411
- JP-A- 2008 516 698
- JP-A- 2011 015 966
- JP-A- 2013 154 166
- US-A1- 2010 012 703
- US-A1- 2014 012 317
- US-A1- 2015 164 503
- US-A1- 2017 189 159
- US-A1- 2018 214 201

## Description

### Technical Field

The present invention relates to an adhesion promotion device.

US 2015/0164503 A1 relates to an apparatus for joining two hollow organ sections with an annular array of surgical staples includes a staple cartridge, an anvil, a buttress member, and a buttress mount.

US 2010/0012703 Al relates to an annular gasket for use with a circular stapling apparatus in performing an anastomosis. The annular gasket comprises a substantially circular body portion having an upper surface, a lower surface, an outer terminal edge, and an inner terminal edge defining an aperture therein.

EP 1647231 Al relates to an apparatus for forming an anastomosis between adjacent intestinal sections of tissue. The apparatus includes an anastomosis device having an anvil and a tubular body portion, wherein the anvil is selectively attachable to the tubular body portion by a shaft, and a support structure for deposition between the intestinal sections of tissue.

In a medical field, a medical procedure (for example, anastomosis for a digestive tract) of joining biological organs to each other by performing a surgical operation is known. In a case where the medical procedure as described above is performed, as a prognosis determinant after surgery, a fact is important that there is no delay in adhesion in a joint portion joined between the biological organs.

In the medical procedure of joining the biological organs, various methods and various medical instruments are used. For example, a method of suturing the biological organs by using a biodegradable suture, or a method of using a mechanical anastomosis device (refer to PTL 1) for suturing the biological organs by using a stapler has been proposed. In particular, in a case where anastomosis is performed using the mechanical anastomosis device, compared to a method of using the suture, a joining force between the biological organs can be improved in the joint portion. Accordingly, risk factors of an anastomotic leakage can be reduced.

### Citation List

### Patent Literature

PTL 1: JP-T-2007-505708

### Summary of Invention

### Technical Problem

However, a progress degree of the adhesion in the joint portion depends on a state of biological tissues in a joint object site (joint target site) of a patient. Therefore, for example, even in a case where the anastomosis device as disclosed in PTL 1 is used, depending on the state of the biological tissues of the patient, there is a possibility that the risk factors of the anastomotic leakage cannot be sufficiently reduced.

Therefore, the present invention aims to provide an adhesion promotion device capable of reducing risk factors of an anastomotic leakage after a surgical operation is performed.

### Solution to Problem

According to an embodiment of the present invention, there is provided an adhesion promotion device according to claim 1. Preferred embodiments are disclosed in the dependent claims.

### Advantageous Effects of Invention

According to the present invention, an adhesion promotion device can improve rigidity of a main body portion by a reinforcement portion, and can prevent distortion or misalignment from occurring. In this manner, it is possible to prevent the distortion or the misalignment from occurring in the adhesion promotion device during an operation (when the adhesion promotion device indwells a body). In addition, in a case where any force is applied after indwelling, it is possible to prevent the distortion or the misalignment from occurring in the adhesion promotion device. Therefore, it is possible to reduce risk factors of an anastomotic leakage after a surgical operation is performed.

### Brief Description of Drawings

[Fig. 1] Fig. 1(A) is a perspective view illustrating a form of an adhesion promotion device of the present invention, and Fig. 1(B) is a perspective view illustrating a main body portion of the adhesion promotion device illustrated in Fig. 1(A).
[Fig. 2] Fig. 2(A) is a cross-sectional view taken along line 2-2 in Fig. 1(A), and Fig. 2(B) is an enlarged cross-sectional view illustrating a portion of the main body portion.
[Fig. 3] Figs. 3(A) and 3(B) are explanatory views used for describing conditions for specifying a shape of a reinforcement portion in the adhesion promotion device.
[Fig. 4] Figs. 4(A), 4(B), and 4(C) are views illustrating various forms of the shape of the reinforcement portion in the adhesion promotion device. The examples shown in Fig. 4(B) and 4(C) do not form part of the invention.
[Fig. 5] Figs. 5(A) and 5(B) are views illustrating various forms of the shape of the reinforcement portion in the adhesion promotion device, subsequently to Fig. 4.
[Fig. 6] Figs. 6(A) and 6(B) are views illustrating various forms of the shape of the reinforcement portion in the adhesion promotion device, subsequently to Fig. 5. The examples shown in Fig. 5(A) and 5(B) do not form part of the invention.
[Fig. 7] Figs. 7(A) and 7(B) are views illustrating a form of a hole portion formed in a main body portion of the adhesion promotion device.
[Fig. 8] Fig. 8 is a perspective view illustrating another form of the adhesion promotion device not forming part of the present invention.
[Fig. 9] Fig. 9(A) is a perspective view illustrating still another form of the adhesion promotion device not forming part of the present invention, and Fig. 9(B) is a perspective view illustrating the main body portion of the adhesion promotion device illustrated in Fig. 9(A).
[Fig. 10] Figs. 10(A), 10(B), and 10(C) are views illustrating another form of the shape of the main body portion in the adhesion promotion device.
[Fig. 11] Figs. 11(A) and 11(B) are cross-sectional views illustrating another form of a cross-sectional structure of the adhesion promotion device.
[Fig. 12] Figs. 12(A) and 12(B) are views illustrating a form of a manufacturing procedure of the reinforcement portion in the adhesion promotion device.
[Fig. 13] Figs. 13(A), 13(B), 13(C), and 13(D) are views illustrating a specific shape of the reinforcement portion in the adhesion promotion device. The examples shown in Fig. 13(A), 13(B) and 13(C) do not form part of the invention.
[Fig. 14] Figs. 14(A), 14(B), 14(C), and 14(D) are views illustrating a specific shape of the reinforcement portion in the adhesion promotion device, subsequently to Fig. 13.
[Fig. 15] Figs. 15(A), 15(B), 15(C), and 15(D) are views illustrating a specific shape of the reinforcement portion in the adhesion promotion device, subsequently to Fig. 14.
[Fig. 16] Fig. 16 is a flowchart illustrating each procedure of a treatment method of using the adhesion promotion device.
[Fig. 17] Fig. 17 is a flowchart illustrating a procedure of an embodiment (large intestine anastomosis) of the treatment method.
[Fig. 18] Fig. 18 is a schematic cross-sectional view for describing the large intestine anastomosis.
[Fig. 19] Fig. 19 is a schematic cross-sectional view for describing the large intestine anastomosis.
[Fig. 20] Fig. 20 is a schematic cross-sectional view for describing the large intestine anastomosis.

### Description of Embodiments

Hereinafter, an embodiment of the present invention will be described with reference to each drawing.
Dimensional proportions in the drawings are exaggerated and different from actual proportions for convenience of description, in some cases.

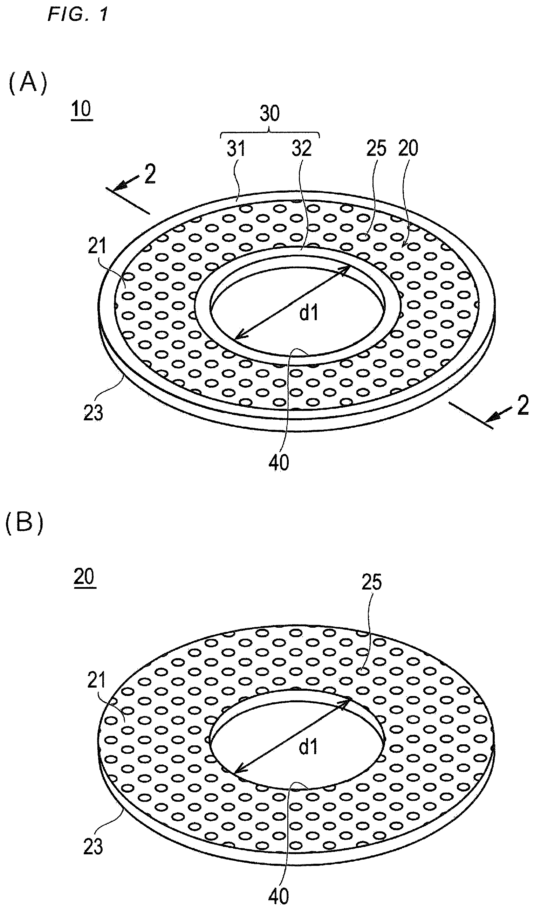

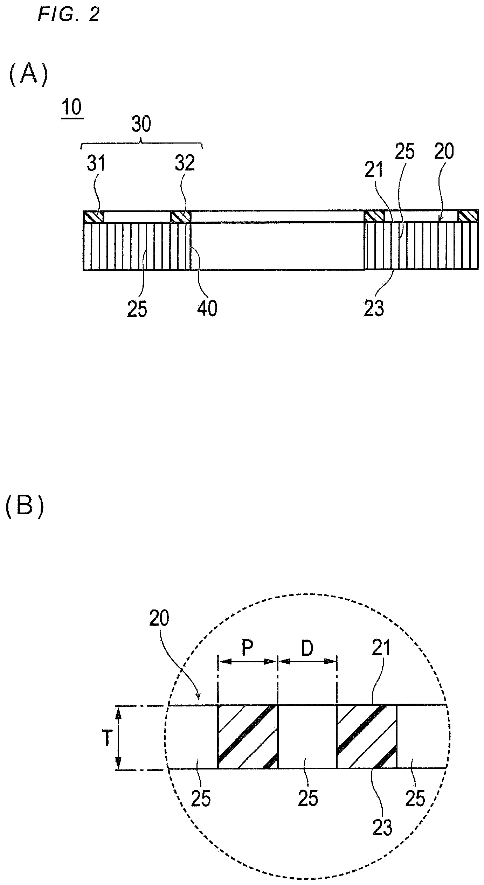

Fig. 1(A) is a perspective view illustrating a form of an adhesion promotion device 10, and Fig. 1(B) is a perspective view illustrating a main body portion 20 of the adhesion promotion device 10 illustrated in Fig. 1(A). Fig. 2(A) is a cross-sectional view taken along line 2-2 in Fig. 1(A), and Fig. 2(B) is an enlarged cross-sectional view illustrating a portion of the main body portion 20.

For example, the illustrated adhesion promotion device 10 can be used for a method of joining a predetermined site serving as a joint object of a biological organ (for example, anastomosis of a digestive tract). In general, the adhesion promotion device 10 has the main body portion 20 formed of a biodegradable sheet having a plurality of through-holes 25 and promoting adhesion of biological tissues, and a reinforcement portion 30 disposed in a portion of the main body portion 20 and reinforcing the main body portion 20. Hereinafter, configurations will be described in detail.

### <Main Body Portion 20>

The main body portion 20 can be formed of the biodegradable sheet having a thin film shape. The main body portion 20 has a plurality of the through-holes 25. As illustrated in Figs. 1(A) and 1(B), the plurality of through-holes 25 are regularly and cyclically provided in a plane direction of the main body portion 20. For example, the plurality of through-holes 25 may be randomly provided in the main body portion 20.

As illustrated in Fig. 2(B), the plurality of through-holes 25 are vertically provided along a thickness direction (upward-downward direction in Fig. 2(B)) of the main body portion 20. The plurality of through-holes 25 are substantially vertically provided between a front surface 21 and a rear surface 23 of the main body portion 20. However, the plurality of through-holes 25 may be provided to be curved between the front surface 21 and the rear surface 23 in the thickness direction of the main body portion 20.

A thickness of the main body portion 20 (size T illustrated in Fig. 2(B)) is not particularly limited. The thickness is preferably 0.05 to 0.3 mm, and more preferably 0.1 to 0.2 mm. When the thickness of the main body portion 20 is 0.05 mm or thicker (particularly, 0.1 mm or thicker), sufficient strength can be ensured to such an extent that the main body portion 20 is not damaged when the adhesion promotion device 10 is handled. On the other hand, when the thickness of the main body portion 20 is 0.3 mm or thinner (particularly, 0.2 mm or thinner), the main body portion 20 closely adheres to a biological tissue to which the main body portion 20 is applied, and it is possible to ensure sufficient flexibility to follow the biological tissue.

As illustrated in Figs. 1(A) and 1(B), the main body portion 20 has a circular shape in a plan view. However, an outer shape of the main body portion 20 is not particularly limited, and may be a substantially rectangular shape or an elliptical shape, for example.

In the main body portion 20, a ratio value of a hole diameter D (distance D illustrated in Fig. 2(B)) of the plurality of through-holes 25 to a pitch P (distance P illustrated in Fig. 2(B)) of the plurality of through-holes 25 is preferably 0.25 or greater and smaller than 40. When a shape of the through-hole 25 is a perfect circle in a plan view, the hole diameter D of the through-hole 25 is equal to a diameter of the perfect circle. On the other hand, in a case where the through-hole 25 is not the perfect circle in a plan view, the diameter (equivalent circle diameter) of the perfect circle having an area the same as an area of an opening portion (portion facing the front surface 21 or the rear surface 23 in the through-hole 25) of the through-hole 25 can be set as the hole diameter D of the through-hole 25.

In addition, the main body portion 20 has the plurality of through-holes 25. Therefore, the main body portion 20 has a plurality of values of the hole diameters D corresponding to the respective through-holes 25. In the present embodiment, in calculating the above-described ratio value, an arithmetic average value of two or more values of the hole diameter D corresponding to each of the plurality of through-holes 25 is used as a representative value of the hole diameter D. On the other hand, the "pitch P" of the plurality of through-holes 25 means a shortest distance between the opening portions of the two through-holes 25. With regard to the value of the pitch P, there are a plurality of values of the pitch P corresponding to a combination of the through-holes 25 adjacent to each other. Therefore, according to the present embodiment, in calculating the above-described ratio value, the arithmetic average value of two or more values of the pitch P corresponding to each combination of the through-holes 25 adjacent to each other is used as a representative value of the pitch P.

The pitch P of the above-described through-holes 25, the hole diameter D, and the ratio of the hole diameter D to the pitch P are merely examples, and the present invention is not limited thereto.

The main body portion 20 can be formed of a biodegradable material. A configuration material of the main body portion 20 is not particularly limited. For example, a biodegradable resin may be used. As the biodegradable resin, for example, it is possible to use a known biodegradable (co)polymer such as those disclosed in JP-T-2011-528275, JP-T-2008-514719, Pamphlet of International Publication No. 2008-1952, and JP-T-2004-509205. Specifically, the biodegradable resin includes (1) a polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose; or (2) copolymer configured to include one or more monomers configuring the above-described materials (1). That is, it is preferable that the biodegradable sheet includes the polymer selected from a group formed of aliphatic polyester, polyester, polyanhydride, polyorthoester, polycarbonate, polyphosphazene, polyphosphate ester, polyvinyl alcohol, polypeptide, polysaccharide, protein, and cellulose, and at least one biodegradable resin selected from a group formed of the copolymer configured to include one or more monomers configuring the polymer.

A manufacturing method of the main body portion 20 is not particularly limited. For example, the manufacturing method includes a method of preparing a fiber formed of the above-described biodegradable resin and manufacturing a mesh-shaped sheet by using the fiber. A method of preparing the fiber formed of the biodegradable resin is not particularly limited. For example, the method includes an electrospinning method (electric field spinning method and electrostatic spinning method) or a melt blowing method. As the method for the main body portion 20, only one of the above-described methods may be selected and used. Alternatively, two or more methods may be used in appropriate combination with each other.

Here, the electrospinning method is a method of forming fine fibers formed of a resin in a state where a high voltage (for example, approximately 20 kV) is applied between a syringe filled with a resin solution and a collector electrode. When the method is adopted, the solution extruded from the syringe is charged and scattered in an electric field. However, a solvent contained in the scattered solution evaporates with the lapse of time. Accordingly, as a result, a thinned solute appears. The thinned solute becomes fine fibers formed of the resin, and adheres to a collector of basal lamellas.

A mesh-shaped base material formed of stainless steel (SUS) is used as the collector of the electrospinning method. In this manner, the fine fibers formed of the biodegradable resin which serves as the thinned solute adhere to a substantial portion of a mesh, thereby forming the mesh formed of the fine fibers. The biodegradable sheet can be manufactured by separating the resin mesh obtained in this way from the mesh-shaped base material. A size (a hole diameter or a pitch) of the mesh-shaped base material is appropriately adjusted. In this manner, it is possible to control a shape (a hole diameter or a pitch of the through-hole) of the biodegradable sheet formed of the manufactured resin mesh.

In addition, as another example of the manufacturing method of similarly using the electrospinning method, the above-described solution is scattered on a front surface of a flat base material having no mesh shape, and the fine fibers are adhered thereto. In this manner, it is possible to adopt a method of forming the through-hole after obtaining the resin sheet having a uniform thickness. In this case, for example, the resin sheet is irradiated with a laser beam focused by using a condenser lens. In this manner, the through-hole can be formed in an irradiation site. Then, energy or an irradiation time of the laser beam to be used for the irradiation, and an interval between the irradiation sites are adjusted. In this manner, it is possible to control the shape (the hole diameter or the pitch of the through-hole) of the biodegradable sheet formed of the manufactured resin mesh.

As still another example of the manufacturing method of the main body portion 20, a fiber formed of the above-described biodegradable resin may be spun in accordance with a usual method, and the obtained fiber may be knitted into a mesh shape to manufacture the biodegradable sheet.

The main body portion 20 causes a biological reaction by using the configuration materials such as the biodegradable resin configuring the main body portion 20. Due to this action, the main body portion 20 induces expression of biological components such as fibrin. The biological components induced in this way can promote adhesion by being accumulated to penetrate the through-holes 25 of the main body portion 20. For example, the main body portion 20 of the adhesion promotion device 10 is disposed between the biological organs serving as the joint object (anastomosis object), thereby promoting the adhesion by using the above-described mechanism.

The main body portion 20 has a hole portion 40 (center hole) whose hole diameter d1 is larger than that of the through-hole 25 at a substantially central position when viewed in a plan view. For example, the hole diameter of the hole portion 40 can be formed to be 5 mm to 25 mm. In addition, an outer shape of the hole portion 40 can be a perfect circle, for example. However, the hole portion 40 may have an elliptical shape, a rectangular shape, or other shapes.

### <Reinforcement Portion 30>

As illustrated in Figs. 1(A) and 2(A), the reinforcement portion 30 is disposed in a portion of the main body portion 20. The reason that the reinforcement portion 30 is disposed in a portion of the main body portion 20 is as follows. In a case where the reinforcement portion 30 is provided on an entire surface of the main body portion 20, a function of promoting the adhesion which is an original function of the main body portion 20 cannot be achieved. The reinforcement portion 30 is disposed on one surface (front surface 21) out of both the front surface and the rear surface of the main body portion 20. Since the main body portion 20 is formed of the mesh sheet, the main body portion 20 is extremely soft. In a case of using only the main body portion 20, distortion or misalignment is likely to occur in the adhesion promotion device. Therefore, there is a possibility that the distortion or the misalignment may occur in the adhesion promotion device during an operation (when the adhesion promotion device indwells a body). In addition, even after the indwelling, there is a possibility that the distortion or the misalignment may occur in the adhesion promotion device due to some reasons.

Therefore, in the adhesion promotion device 10 of the present embodiment, rigidity of the main body portion 20 is improved by disposing the reinforcement portion 30 in a portion of the main body portion 20. Accordingly, it is possible to prevent the distortion or the misalignment from occurring in the adhesion promotion device 10. In this manner, it is possible to prevent the distortion or the misalignment from occurring in the adhesion promotion device 10 during the operation (when the adhesion promotion device 10 indwells a body). In addition, in a case where any force is applied after the indwelling, it is possible to prevent the distortion or the misalignment from occurring in the adhesion promotion device 10.

The reinforcement portion 30 includes a first reinforcement portion 31 located on an outer edge side of the main body portion 20 and a second reinforcement portion 32 located on an inner peripheral edge side of the hole portion 40. In the main body portion 20, the rigidity of the outer edge side is improved by the first reinforcement portion 31, and the rigidity of the inner peripheral edge side of the hole portion 40 is improved by the second reinforcement portion 32. When a medical instrument is inserted into the hole portion 40, it is possible to prevent the distortion or the misalignment from occurring in the adhesion promotion device 10.

The reinforcement portion 30 is not limited to a case where the two first and second reinforcement portions 31 and 32 are provided. One reinforcement portion 30 can be located only on the outer edge side of the main body portion 20, or can be located only on the inner peripheral edge side of the hole portion 40. In addition, the reinforcement portion 30 can be configured to include three or more reinforcement portions 30.

The second reinforcement portion 32 can be stronger than the first reinforcement portion 31 in reinforcing the biodegradable sheet forming the main body portion 20. Since the rigidity of the inner peripheral edge side of the hole portion 40 is further improved, it is possible to further prevent the distortion or the misalignment from occurring in the adhesion promotion device 10 when a medical instrument is inserted into the hole portion 40.

The first reinforcement portion 31 and the second reinforcement portion 32 can have the same strength in reinforcing the main body portion 20.

A material of the reinforcement portion 30 is not particularly limited. However, for example, a biocompatible adhesive or a coating agent formed of a thermoplastic resin can be used. In addition, a material the same as that of the main body portion 20 can be used.

The thickness of the reinforcement portion 30 is not particularly limited. The reinforcement portion 30 is provided in order to reinforce the main body portion 20, and the reinforcement portion 30 does not need to be flexible or elastic. Therefore, the reinforcement portion 30 does not need to be thicker than necessary, and for example, an intended purpose can be achieved by the thickness of smaller than 1 mm.

A preparation method of the reinforcement portion 30 is not particularly limited, and a preparation method suitable for the configuration material of the reinforcement portion 30 can be adopted. For example, in a case of using the biocompatible adhesive or the coating agent, the reinforcement portion 30 can be prepared by applying the biocompatible adhesive or the coating agent from a nozzle to a portion of the main body portion 20 and drying the applied portion. In addition, a layer including the reinforcement portion 30 is formed separately from the main body portion 20, and a reinforcement portion layer thereof is integrated with the main body portion 20 by means of crimping or heat-welding. In this manner, the reinforcement portion can be prepared.

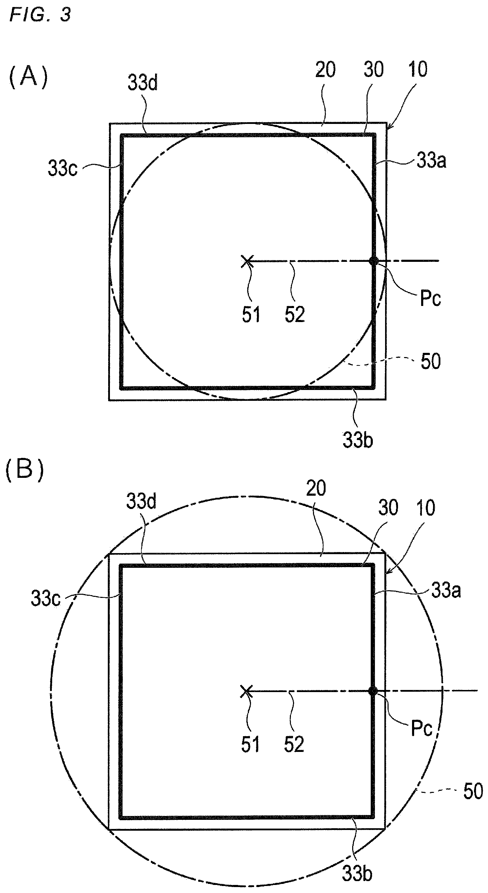

Conditions for specifying the shape of the reinforcement portion 30 will be described with reference to Figs. 3(A) and 3(B).

In the adhesion promotion device 10 illustrated in Figs. 3(A) and 3(B), the main body portion 20 and the reinforcement portion 30 have a quadrangular shape. For convenience of description, sides configuring the reinforcement portion 30 having the quadrangular shape are defined as 33a to 33d. First, a virtual circle 50 (Fig. 3(A)) inscribed with the main body portion 20 or a virtual circle 50 (Fig. 3(B)) circumscribed with the main body portion 20 is assumed. It is preferable that the reinforcement portion 30 intersects with a line segment 52 extending from a center 51 of the assumed virtual circle 50 at least at one location. In the illustrated example, the line segment 52 extending from the center 51 of the virtual circle 50 and the side 33a of the reinforcement portion 30 intersect with each other at a point Pc. In a case of adopting this form, the rigidity of the main body portion 20 is improved by the side 33a of the reinforcement portion 30. For example, even in a case where a force F of pushing down or pulling up the main body portion 20 in a direction orthogonal to a paper surface of the drawing is applied to the point Pc, the side 33a of the reinforcement portion 30 is less likely to be bent. Therefore, in the adhesion promotion device 10, the distortion or the misalignment is prevented from occurring against the force F.

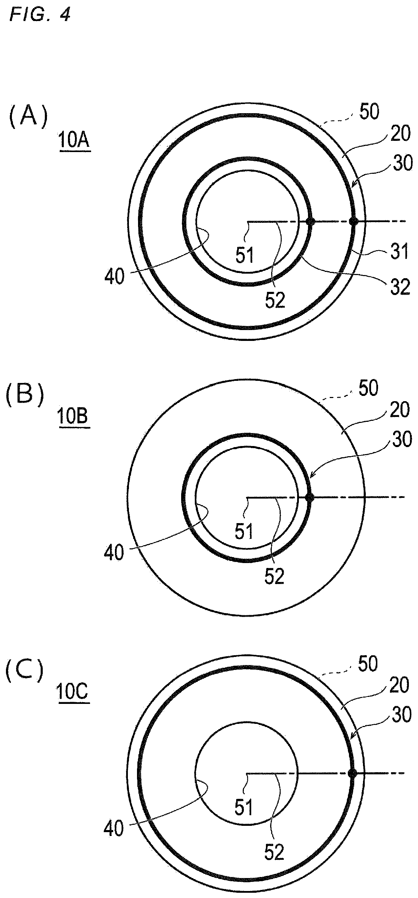

Various forms of the shape of the reinforcement portion 30 in the adhesion promotion device will be described with reference to Figs. 4(A), 4(B), 4(C), 5(A), 5(B), 6(A), and 6(B).

The reinforcement portion 30 in an adhesion promotion device 10A in Fig. 4(A) includes the first reinforcement portion 31 located on the outer edge side of the main body portion 20 and the second reinforcement portion 32 located on the inner peripheral edge side of the hole portion 40. Both the first reinforcement portion 31 and the second reinforcement portion 32 have a closed ring shape.

The reinforcement portion 30 in an adhesion promotion device 10B in Fig. 4(B) is located only on the inner peripheral edge side of the hole portion 40, and has a closed ring shape.

The reinforcement portion 30 in an adhesion promotion device 10C in Fig. 4(C) is located only on the outer edge side of the main body portion 20, and has a closed ring shape.

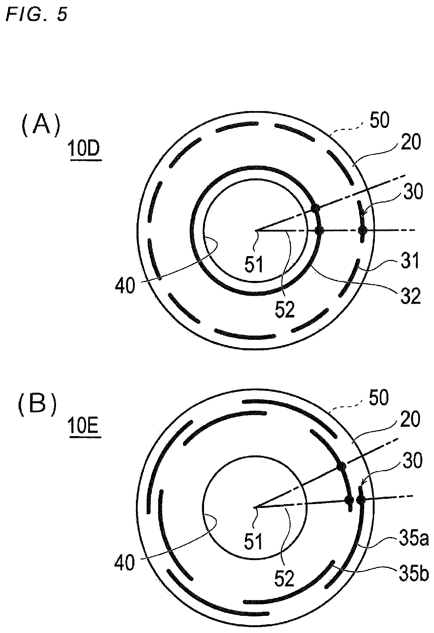

The reinforcement portion 30 in an adhesion promotion device 10D in Fig. 5(A) includes the first reinforcement portion 31 located on the outer edge side of the main body portion 20 and the second reinforcement portion 32 located on the inner peripheral edge side of the hole portion 40. The first reinforcement portion 31 extends in a circular shape with a gap, and has an open ring shape. The second reinforcement portion 32 has a closed ring shape.

The reinforcement portion 30 in an adhesion promotion device 10E in Fig. 5(B) includes two reinforcement portions 35a and 35b located close to the outer edge side of the main body portion 20. The two reinforcement portions 35a and 35b are concentrically located, and both have an open ring shape. When viewed from the center 51 of the virtual circle 50, a gap in the reinforcement portion 35a located outside in the radial direction is hidden by the reinforcement portion 35b located inside in the radial direction. On the other hand, when viewed from the center 51 of the virtual circle 50, a gap in the reinforcement portion 35b located inside in the radial direction is hidden by the reinforcement portion 35a located outside in the radial direction.

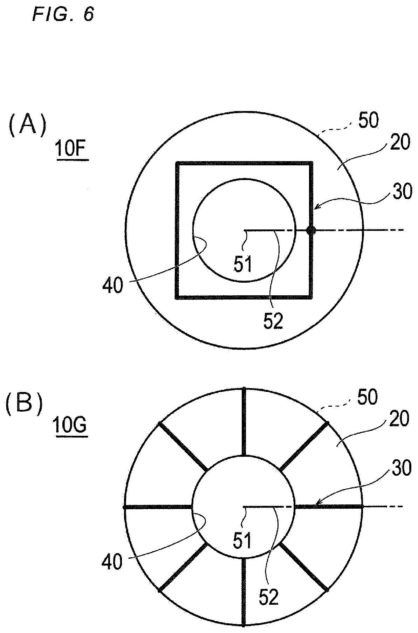

The reinforcement portion 30 in an adhesion promotion device 10F in Fig. 6(A) has a closed rectangular shape.

The reinforcement portion 30 in an adhesion promotion device 10G in Fig. 6(B) has a radial shape extending in the radial direction from the center 51 of the virtual circle 50.

All of the reinforcement portions 30 are disposed in a portion of the main body portion 20. Therefore, all of the through-holes 25 are not blocked, and the main body portion 20 can sufficiently achieve a function of promoting the adhesion which is an original function.

The reinforcement portion 30 illustrated in Fig. 4(A) intersects with the line segment 52 extending from the center 51 of the virtual circle 50 inscribed or circumscribed with the main body portion 20 at two locations. The reinforcement portion 30 illustrated in Figs. 4(B) and 4(C) intersects with the line segment 52 at one location. The reinforcement portion 30 illustrated in Figs. 5(A) and 5(B) intersects with the line segment 52 at two locations in one direction of the line segment 52, and intersects with the line segment 52 at one location in the other direction. The reinforcement portion 30 illustrated in Fig. 6(A) intersects with the line segment 52 at one location. The reinforcement portion 30 illustrated in Fig. 6(B) extends on the line segment 52, and a width direction of the reinforcement portion 30 is a direction intersecting with the line segment 52. Therefore, all of the reinforcement portions 30 intersect with the line segment 52 extending from the center 51 of the virtual circle 50 at least at one location. Since the main body portion 20 has a circular shape, the virtual circle 50 coincides with an outer periphery of the main body portion 20.

Except for the reinforcement portion 30 having a radial shape illustrated in Fig. 6(B), the reinforcement portions 30 illustrated in Figs. 4(A), 4(B), 4(C), 5(A), 5(B), and 6(A) intersect with the line segment 52 over the entire periphery of the virtual circle 50 in a circumferential direction. That is, in a case where the line segment 52 is rotated once (360 degrees) around the center 51 of the virtual circle 50, the reinforcement portion 30 intersects with the line segment 52 in any direction. In other words, the reinforcement portions 30 have a shape that forms a closed region internally including the center 51 of the virtual circle 50. Since the reinforcement portion 30 intersects with the line segment 52 over the entire periphery of the virtual circle 50 in the circumferential direction, the reinforcement portion 30 is less likely to be bent even when the force F is applied along any direction. Therefore, the adhesion promotion device 10 can prevent the distortion or the misalignment from occurring against the force F applied along any direction.

The reinforcement portion 30 illustrated in Figs. 5(A) and 5(B) includes a portion having an open ring shape. However, all of the reinforcement portions 30 have a shape overlapping in an extending direction of the line segment 52. Even when the portion having this open ring shape is included, the reinforcement portion 30 intersecting with the line segment 52 can be formed over the entire periphery of the virtual circle 50 in the circumferential direction.

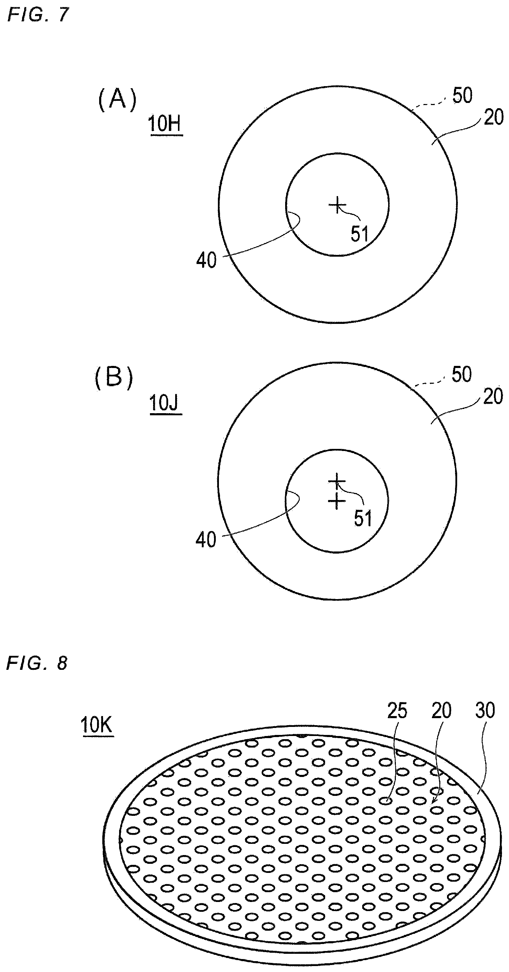

A form of the hole portion 40 formed in the main body portion 20 of adhesion promotion devices 10H and 10J will be described with reference to Figs. 7(A) and 7(B).

The hole portion 40 illustrated in Fig. 7(A) is a center hole formed concentrically with the center 51 of the virtual circle 50 inscribed or circumscribed with the main body portion 20. The hole portion 40 illustrated in Fig. 7(B) is formed at an eccentric position from the center 51 of the virtual circle 50. In this way, the hole portion 40 can be formed at a desired position in the main body portion 20.

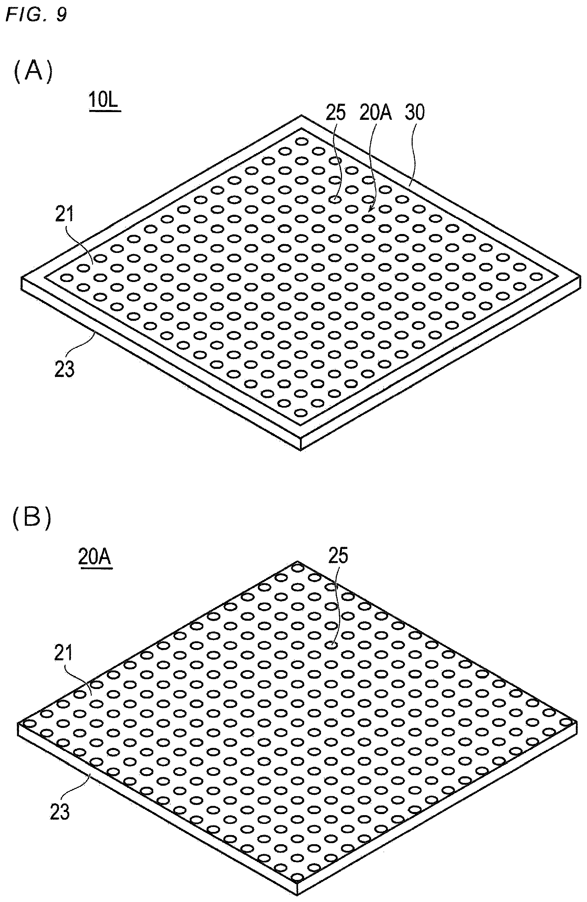

Another form of the adhesion promotion device will be described with reference to Figs. 8, 9(A), and 9(B).

As illustrated in Figs. 8, 9(A), and 9(B), adhesion promotion devices 10K and 10L may not have the hole portion 40 formed in the main body portion 20.

The main body portion 20 is not limited to a case having a circular shape in a plan view. As illustrated in Figs. 9(A) and 9(B), an outer shape of a main body portion 20A can be a substantially rectangular shape.

The reinforcement portion 30 in the adhesion promotion device 10K illustrated in Fig. 8 is located only on the outer edge side of the main body portion 20, and has a closed ring shape. The reinforcement portion 30 in the adhesion promotion device 10L illustrated in Fig. 9(A) is located only on the outer edge side of the main body portion 20A, and has a closed and substantially rectangular shape.

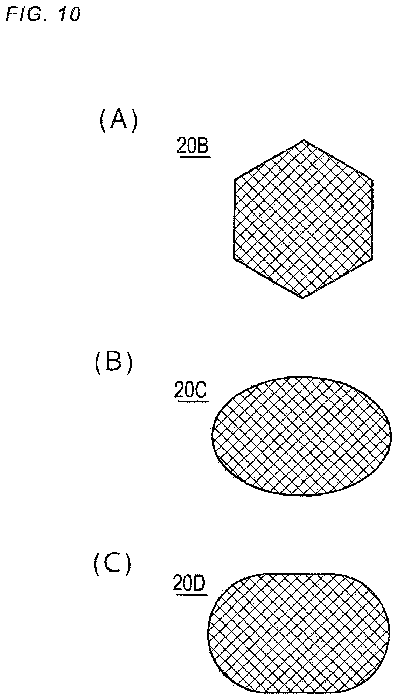

Another form of the shape of the main body portion in the adhesion promotion device will be described with reference to Figs. 10(A), 10(B), and 10(C).

The outer shape of the main body portions 20 and 20A is not limited to the above-described circular shape or substantially rectangular shape. The outer edge of the main body portion can have various shapes including a linear shape or an arc shape. For example, as illustrated in Fig. 10(A), an outer edge of a main body portion 20B can have a polygonal shape (hexagonal shape in the illustrated example) including the linear shape. In addition, as illustrated in Fig. 10(B), an outer edge of a main body portion 20C can have an elliptical shape including an arc shape. In addition, as illustrated in Fig. 10(C), an outer edge of a main body portion 20D can have a track-like shape including both the linear shape and the arc shape.

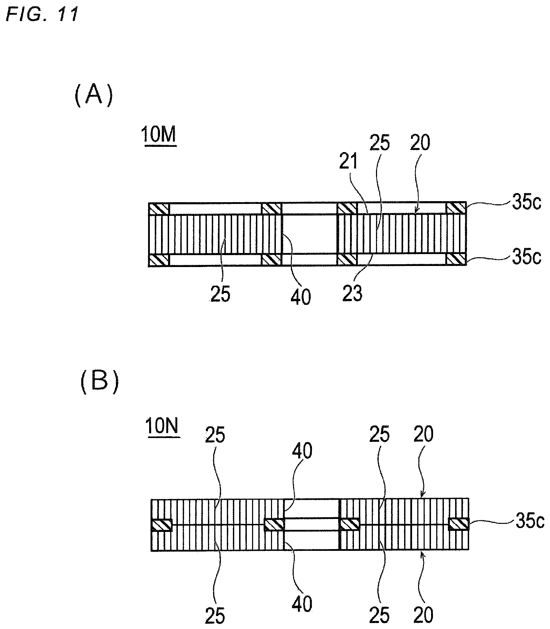

Another form of a cross-sectional structure of the adhesion promotion device will be described with reference to Figs. 11(A) and 11(B).

The cross-sectional structure of the adhesion promotion device is not limited to a structure in which a layer including the reinforcement portion 30 (reinforcement portion layer) is integrated with one side of the main body portion 20 (refer to Fig. 2(A)).

For example, as illustrated in Fig. 11(A), an adhesion promotion device 10M can have a cross-sectional structure in which a reinforcement portion layer 35c is integrated with both the front and rear surfaces 21 and 23 of the main body portion 20. In addition, as illustrated in Fig. 11(B), an adhesion promotion device 10N can have a cross-sectional structure in which the main body portion 20 is integrated with both surfaces of the reinforcement portion layer 35c. The reinforcement portion layer 35c and the main body portion 20 can be integrated by means of crimping or heat-welding.

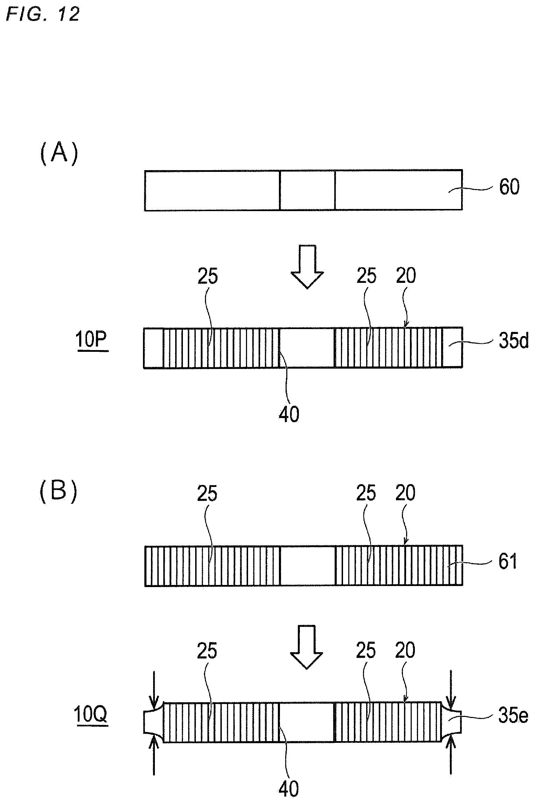

A form of a manufacturing procedure of the reinforcement portion in adhesion promotion devices 10P and 10Q will be described with reference to Figs. 12(A) and 12(B).

The reinforcement portion 30 in the adhesion promotion device 10 is not limited to a case where the reinforcement portion 30 is manufactured by integrating the layer including the reinforcement portion (reinforcement portion layer) and the main body portion 20 with each other.

For example, as illustrated in Fig. 12(A), a biodegradable sheet material 60 before forming the through-hole 25 is prepared. Then, the through-hole 25 is formed in the biodegradable sheet material 60 to form the main body portion 20, except for a region configuring a reinforcement portion 35d. Whereas the main body portion 20 has the plurality of through-holes 25, the through-hole 25 is no formed in the reinforcement portion 35d. Therefore, the reinforcement portion 35d of the adhesion promotion device 10P has rigidity higher than that of the main body portion 20, and can reinforce the main body portion 20.

In addition, as illustrated in Fig. 12(B), a biodegradable sheet 61 having the plurality of through-holes 25 is prepared. Then, only a region configuring a reinforcement portion 35e is compressed or heated in a thickness direction to crush the through-hole 25. The through-hole 25 in the reinforcement portion 35e does not need to be completely extinguished. The through-holes 25 may be crushed and reduced, and configuration materials of the biodegradable sheet may be in a densely aggregated form. Whereas the main body portion 20 has the plurality of through-holes 25, the through-holes 25 in the reinforcement portion 35e are crushed and reduced. Therefore, the reinforcement portion 35e of the adhesion promotion device 10Q has higher density. Accordingly, the rigidity is improved compared to the main body portion 20, and the main body portion 20 can be reinforced.

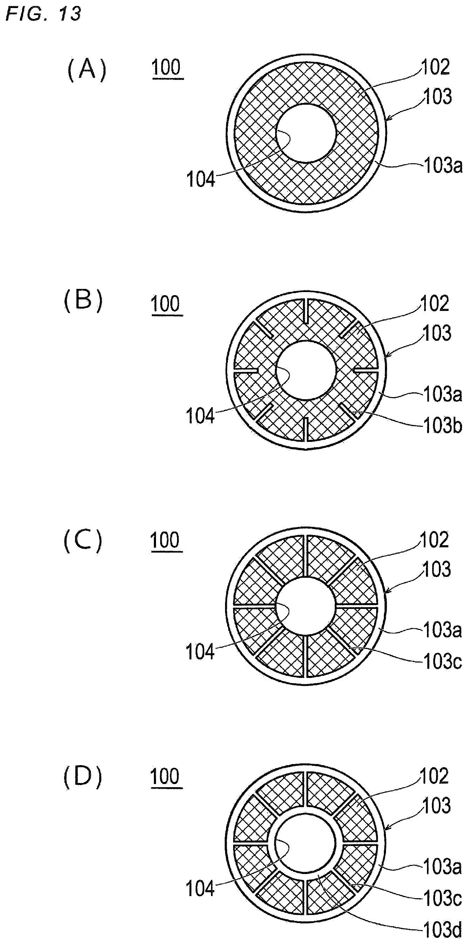

A specific shape of a reinforcement portion in an adhesion promotion device 100 will be described with reference to Figs. 13 to 15.

The reinforcement portion can have any desired shape including the linear shape, or the arc shape, as long as the reinforcement portion is disposed in a portion of the main body portion and achieves a function of reinforcing the main body portion. In all of the adhesion promotion devices 100 illustrated in Figs. 13 to 15, a main body portion 102 has a circular shape in a plan view, and a hole portion 104 (center hole) is formed at a substantially central position when the main body portion 102 is viewed in a plan view.

The reinforcement portion 103 in Fig. 13(A) has an outer ring portion 103a located only on the outer edge side of the main body portion 102 and having a closed shape. The reinforcement portion 103 is configured to include a portion having an arc shape.

The reinforcement portion 103 in Fig. 13(B) has the outer ring portion 103a having the arc shape, and a rib portion 103b extending in the radial direction. The rib portion 103b extends toward the hole portion 104 from an inner periphery of the outer ring portion 103a, but does not reach the hole portion 104. The reinforcement portion 103 is configured to include a portion having the arc shape and the linear shape.

In the reinforcement portion 103 in Fig. 13(C), the rib portion 103c reaches an inner peripheral edge of the hole portion 104, compared to Fig. 13(B).

Compared to Fig. 13(C), the reinforcement portion 103 in Fig. 13(D) has an inner ring portion 103d located on the inner peripheral edge side of the hole portion 104 and having a closed shape. The rib portion 103c connects the outer ring portion 103a and the inner ring portion 103d. The outer ring portion 103a corresponds to a first reinforcement portion 31, and the inner ring portion 103d corresponds to a second reinforcement portion 32.

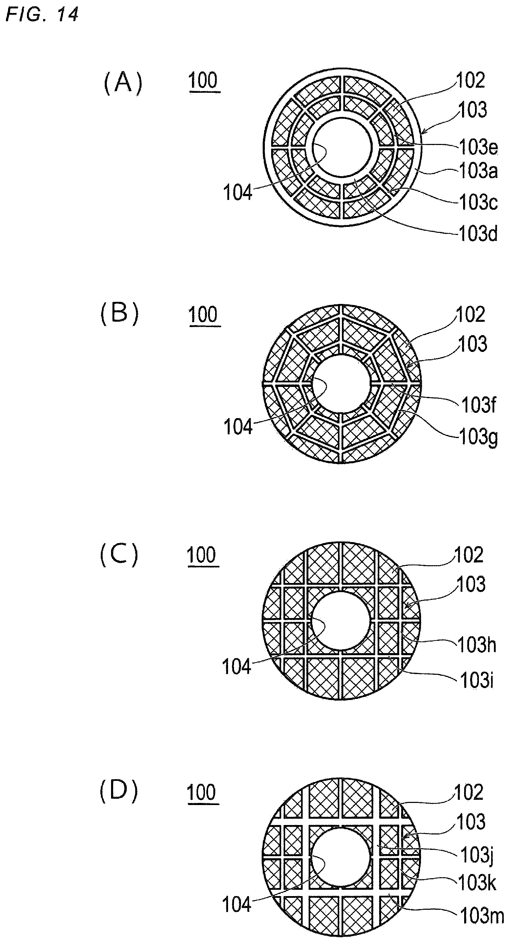

Compared to Fig. 13(D), the reinforcement portion 103 in Fig. 14(A) has an intermediate ring portion 103e located between the outer ring portion 103a and the inner ring portion 103d and having a closed shape. The rib portion 103c connects the outer ring portion 103a, the intermediate ring portion 103e, and the inner ring portion 103d.

The reinforcement portion 103 in Fig. 14(B) is configured to include a portion having the linear shape, and is formed in a shape like a spider web. The reinforcement portion 103 has a rib portion 103f extending in the radial direction, and a connection rib portion 103g connecting the adjacent rib portions 103f to each other. The rib portion 103f extends from the outer edge of the main body portion 102 to the inner peripheral edge of the hole portion 104. The connection rib portion 103g forms an octagonal shape.

The reinforcement portion 103 in Fig. 14(C) is configured to include a portion having the linear shape. The reinforcement portion 103 has a vertical rib portion 103h extending in a vertical direction and a horizontal rib portion 103i extending in a horizontal direction in the drawing. The vertical rib portion 103h and the horizontal rib portion 103i are orthogonal to each other at least at one location. The vertical rib portion 103h and the horizontal rib portion 103i are formed so that width dimensions are substantially equal to each other.

Compared to Fig. 14(C), in the reinforcement portion 103 in Fig. 14(D), vertical rib portions 103j and 103k and a horizontal rib portion 103m have different width dimensions. The width dimension of the vertical rib portion 103j located on the inner peripheral edge side of the hole portion 104 and the width dimension of the horizontal rib portion 103m are larger than the width dimension of the vertical rib portion 103k located on the outer edge side of the main body portion 102. The reinforcement portion 103 is configured to include the first reinforcement portion 31 located on the outer edge side of the main body portion 102 and the second reinforcement portion 32 located on the inner peripheral edge side of the hole portion 104. Then, depending on a size of the width dimension, the second reinforcement portion 32 is stronger than the first reinforcement portion 31 in reinforcing the biodegradable sheet forming the main body portion 102.

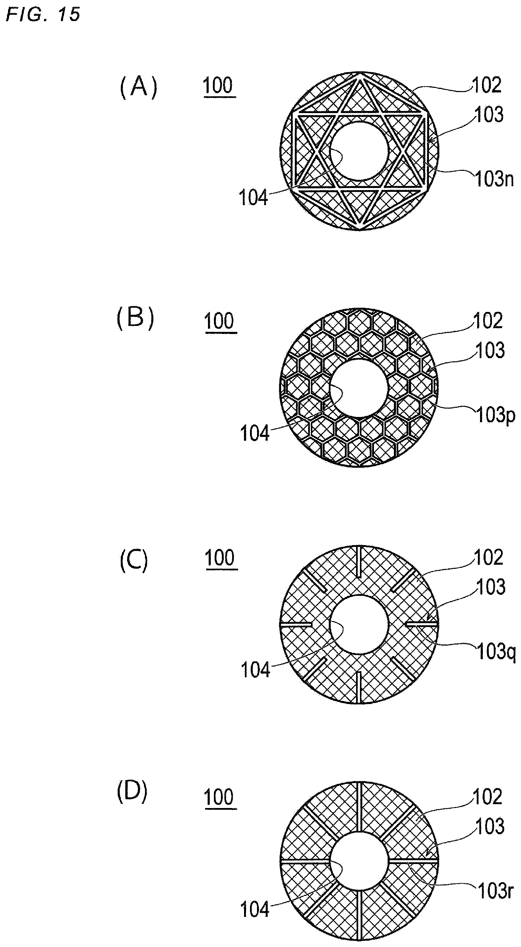

The reinforcement portion 103 in Fig. 15(A) is configured to include a portion having the linear shape. The reinforcement portion 103 is formed in such a way that a plurality of linear rib portions 103n are connected to form a triangular shape, a quadrangular shape, and a hexagonal shape.

The reinforcement portion 103 in Fig. 15(B) is formed in such a way that a plurality of linear rib portions 103p are connected to form a honeycomb structure.

The reinforcement portion 103 in Fig. 15(C) is configured to include a portion having the linear shape. The reinforcement portion 103 has a rib portion 103q extending in the radial direction. The rib portion 103q extends toward the hole portion 104 from the outer edge of the main body portion 102, but does not reach the hole portion 104.

Compared to Fig. 15(C), in the reinforcement portion 103 in Fig. 15(D), a rib portion 103r reaches the inner peripheral edge of the hole portion 104.

In all of the adhesion promotion devices 100 illustrated in Figs. 13 to 15, the rigidity of the main body portion 102 is improved by the reinforcement portion 103, and it is possible to prevent the distortion or the misalignment from occurring in the adhesion promotion device 100.

As described above, the adhesion promotion device 10 has the main body portion 20 formed of the biodegradable sheet having the plurality of through-holes 25 and promoting the adhesion of the biological tissues, and the reinforcement portion 30 disposed in a portion of the main body portion 20 and reinforcing the main body portion 20. According to the adhesion promotion device 10 configured in this way, the rigidity of the main body portion 20 is improved by the reinforcement portion 30, and it is possible to prevent the distortion or the misalignment from occurring in the adhesion promotion device 10. In this manner, it is possible to prevent the distortion or the misalignment from occurring in the adhesion promotion device 10 during the operation (when the adhesion promotion device 10 indwells the body). In addition, in a case where any force is applied after the indwelling, it is possible to prevent the distortion or the misalignment from occurring in the adhesion promotion device 10. Therefore, it is possible to reduce risk factors of an anastomotic leakage after a surgical operation is performed.

The reinforcement portion 30 intersects with the line segment 52 extending from the center 51 of the virtual circle 50 inscribed or circumscribed with the main body portion 20 at least at one location. According to this configuration, even in a case where the force F of bending the reinforcement portion 30 is applied to an intersection portion between the line segment 52 and the reinforcement portion 30, the reinforcement portion 30 is less likely to be bent. Therefore, the adhesion promotion device 10 can prevent the distortion or the misalignment from occurring against the force F.

The reinforcement portion 30 intersects with the line segment 52 over the entire periphery of the virtual circle 50 in the circumferential direction. According to this configuration, the reinforcement portion 30 is less likely to be bent even when the force F is applied along any direction. Therefore, the adhesion promotion device 10 can prevent the distortion or the misalignment from occurring against the force F applied along any direction.

The reinforcement portion 30 has a shape overlapping in an extending direction of the line segment 52. According to this configuration, even in a case where the reinforcement portion 30 has an open shape, it is possible to form the reinforcement portion 30 intersecting with the line segment 52 over the entire periphery of the virtual circle 50 in the circumferential direction. Therefore, the adhesion promotion device 10 can prevent the distortion or the misalignment from occurring against the force F applied along any direction.

The main body portion 20 further has the hole portion 40 formed to have the hole diameter larger than that of the through-hole 25. According to this configuration, it is possible to provide the adhesion promotion device 10 having a shape suitable for treatment using the adhesion promotion device 10.

The reinforcement portion 30 includes the first reinforcement portion 31 located on the outer edge side of the main body portion 20 and the second reinforcement portion 32 located on the inner peripheral edge side of the hole portion 40. According to this configuration, in the main body portion 20, not only the rigidity of the outer edge side can be improved by the first reinforcement portion 31, but also the rigidity of the inner peripheral edge side of the hole portion 40 can be improved by the second reinforcement portion 32. When a medical instrument is inserted into the hole portion 40, it is possible to prevent the distortion or the misalignment from occurring in the adhesion promotion device 10.

In this case, the second reinforcement portion 32 is stronger than the first reinforcement portion 31 in reinforcing the biodegradable sheet forming the main body portion 20. According to this configuration, the rigidity of the inner peripheral edge side of the hole portion 40 can be further improved. When the medical instrument is inserted into the hole portion 40, it is possible to further prevent the distortion or the misalignment from occurring in the adhesion promotion device 10.

The outer edge of the main body portion 20 has the linear shape or the arc shape. According to this configuration, it is possible to provide the adhesion promotion device 10 having a shape suitable for treatment using the adhesion promotion device 10.

The reinforcement portion 30 has the linear shape or the arc shape. According to this configuration, the shape of the reinforcement portion 30 suitable for the shape of the main body portion 20 can be selected, and it is possible to further prevent the distortion or the misalignment from occurring in the adhesion promotion device 10.

Hitherto, the configuration of the adhesion promotion device 10 has been described. However, the specific configuration of the adhesion promotion device 10 is not particularly limited as long as the sheet-like main body portion 20 having a function of promoting the adhesion of the biological tissues is reinforced by the reinforcement portion 30 disposed in a portion of the main body portion 20. As another modification example, for example, the adhesion promotion device 10 may have a communication hole exposed on a side surface of the main body portion 20. In addition, the adhesion promotion device 10 may have a shape partially widened in a direction in which the through-hole 25 formed in the main body portion 20 intersects with the thickness direction (upward-downward direction in Fig. 2(B)) of the main body portion 20. In addition, the adhesion promotion device 10 may have a shape partially narrowed in the direction in which the through-hole 25 formed in the main body portion 20 intersects with the thickness direction (upward-downward direction in Fig. 2(B)) of the main body portion 20.

Next, a treatment method of using the adhesion promotion device will be described.

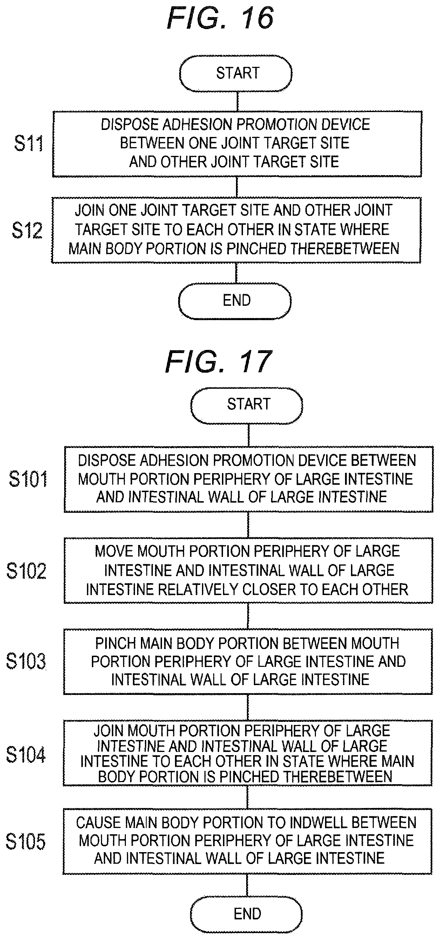

Fig. 16 is a flowchart illustrating each procedure of the treatment method of using the adhesion promotion device.

The treatment method includes disposing an adhesion promotion device provided with a sheet-like main body portion for promoting the adhesion of the biological tissues between one joint target site and the other joint target site which serve as a joint object of the biological organ (S11), and joining the one joint target site and the other joint target site in a state where at least a portion of the main body portion of the adhesion promotion device is disposed between the one joint target site and the other joint target site (S12).

The biological organ and the joint target site in the biological organ which are joined by using the treatment method are not particularly limited, and can be optionally selected. However, in the following description, an example will be described in which the treatment method is applied to large intestine anastomosis. In addition, as the adhesion promotion device used in each medical procedure described below, for example, it is possible to select any desired one from the adhesion promotion devices described above, and it is also possible to select other adhesion promotion devices. However, in the following description, as a representative example which can be preferably used for each medical procedure, an example of using a specific adhesion promotion device will be described. In addition, in each medical procedure described below, detailed description of known medical procedures, known medical devices, and medical instruments will be appropriately omitted.

Hereinafter, in the description herein, "disposing the adhesion promotion device between the biological organs" means at least any one of disposing the adhesion promotion device in a state of being in direct or indirect contact with the biological organs, disposing the adhesion promotion device in a state where a spatial gap is formed with the biological organs, and disposing the adhesion promotion device in both the states (for example, disposing the adhesion promotion device in a state where the adhesion promotion device is in contact with one biological organ and the adhesion promotion device is not in contact with the other biological organ). In addition, in the description herein, a "periphery" does not define a strict range (region), and means a predetermined range (region) as long as a treatment purpose (joining the biological organs to each other) can be achieved. In addition, as long as the treatment purpose can be achieved, in the medical procedure described in the respective treatment methods, orders can be appropriately switched therebetween. In addition, in the description herein, "moving the portions relatively closer to each other" means both moving two or more movement objects closer to each other, and moving only one closer to the other one.

### <Embodiment of Treatment Method (Large Intestine Anastomosis)>

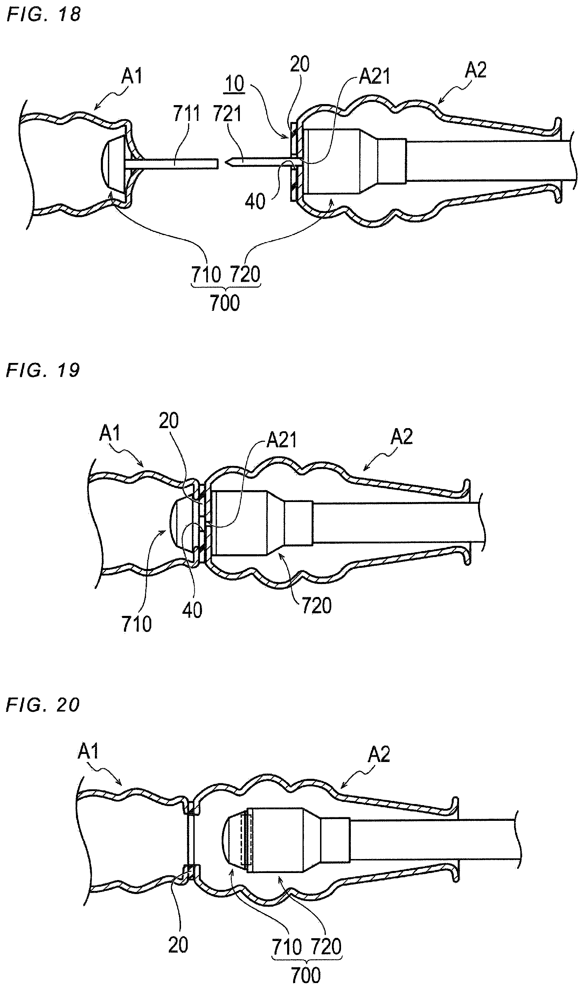

Fig. 17 is a flowchart illustrating a procedure of an embodiment (large intestine anastomosis) of the treatment method, and Figs. 18 to 20 are views for describing the large intestine anastomosis.

In the treatment method according to the present embodiment, the biological organ serving as the joint object is a large intestine cut due to excision of a cancer tumor. Specifically, the biological organs serving as the joint object are a cut mouth side A1 of the large intestine and a cut anal side A2 of the large intestine. In the following description, a joining procedure will be described in which a mouth portion periphery (one joint target site) on the cut mouth side A1 of the large intestine and a portion (the other joint target site) of an intestinal wall on the cut anal side A2 of the large intestine are joined to each other. In addition, in the present embodiment, an example of using the adhesion promotion device 10 illustrated in Fig. 1(A) will be described.

As illustrated in Fig. 17, the treatment method according to the present embodiment includes disposing the adhesion promotion device 10 between the mouth portion periphery of the large intestine and the intestinal wall of the large intestine (S101), moving the mouth portion periphery of the large intestine and the intestinal wall of the large intestine relatively closer to each other (S102), pinching the main body portion of the adhesion promotion device 10 between the mouth portion periphery of the large intestine and the intestinal wall of the large intestine (S103), joining both of these to each other in a state where the main body portion 20 of the adhesion promotion device 10 is pinched between the mouth portion periphery of the large intestine and the intestinal wall of the large intestine (S104), and causing the main body portion of the adhesion promotion device to indwell between the mouth portion periphery of the large intestine and the intestinal wall of the large intestine (S105).

Next, the treatment method according to the present embodiment will be described in detail with reference to Figs. 18 to 20.

As illustrated in Fig. 18, an operator such as a doctor (hereinafter, referred to as an operator) inserts a first engagement instrument 710 of an anastomosis device 700 into the mouth side A1 of the large intestine. The operator disposes a second engagement instrument 720 of the anastomosis device 700 on the anal side A2 of the large intestine. Before the second engagement instrument 720 is disposed on the anal side A2 of the large intestine, the operator forms a through-hole A21 for inserting the second engagement instrument 720 of the anastomosis device 700 into the anal side A2 of the large intestine. A timing at which the through-hole A21 is formed is not particularly limited as long as the timing is before the second engagement instrument 720 is disposed.

For example, as the anastomosis device 700, a known device used for the large intestine anastomosis can be used. As the first engagement instrument 710 and the second engagement instrument 720 engage with each other, the anastomosis device 700 excises the biological tissue disposed between the first engagement instrument 710 and the second engagement instrument 720, and sutures a periphery of the excised biological tissue into a circumferential shape by using a stapler. For example, the first engagement instrument 710 is an instrument including a cylindrical engagement target portion 711. For example, the second engagement instrument 720 is an instrument including an engagement pin 721 to engage with and to be inserted into the engagement target portion 711 of the first engagement instrument 710.

Next, as illustrated in Fig. 18, the operator disposes the adhesion promotion device 10 between the mouth side A1 of the large intestine and the anal side A2 of the large intestine. According to the present embodiment, the adhesion promotion device 10 (refer to Fig. 1(A)) where the hole portion (center hole) 40 is formed in the main body portion 20 is used. When the operator disposes the adhesion promotion device 10, the operator causes the engagement pin 721 included in the second engagement instrument 720 to pass through the hole portion 40 formed in the main body portion 20. In this case, the operator brings the main body portion 20 of the adhesion promotion device 10 into contact with the vicinity having the through-hole A21 formed on the anal side A2 of the large intestine. The operator may dispose the adhesion promotion device 10 on the mouth side A1 of the large intestine by causing the engagement target portion 711 included in the first engagement instrument 710 to pass through the hole portion 40 formed in the main body portion 20.

Next, as illustrated in Fig. 19, the operator engages the first engagement instrument 710 and the second engagement instrument 720 with each other by moving both of these relatively closer to each other. The operator pinches the mouth portion periphery on the mouth side A1 of the large intestine, the main body portion 20 of the adhesion promotion device 10, the periphery of the through-hole A21 formed on the intestinal wall on the anal side A2 of the large intestine between the first engagement instrument 710 and the second engagement instrument 720. The operator operates the anastomosis device 700. In this manner, while the operator excises a portion on the mouth side A1 of the large intestine, a portion of the main body portion 20 of the adhesion promotion device 10, and a portion on the anal side A2 of the large intestine which are pinched between the first engagement instrument 710 and the second engagement instrument 720, the operator joins the peripheries of the excised portions to each other by using a stapler (not illustrated).

Next, as illustrated in Fig. 20, the operator removes the anastomosis device 700 to the outside of the living body from the anal side A2 of the large intestine via an anus, for example. In addition, the operator causes the adhesion promotion device 10 to indwell in a state where a portion of the main body portion 20 of the adhesion promotion device 10 is pinched between the mouth portion periphery on the mouth side A1 of the large intestine and the intestinal wall on the anal side A2 of the large intestine.

As described above, in the treatment method according to the present embodiment, the mouth portion periphery of the large intestine and the intestinal wall of the large intestine are joined to each other. According to the treatment method, the main body portion 20 of the adhesion promotion device 10 disposed between the mouth portion periphery on the mouth side A1 of the large intestine and the intestinal wall on the anal side A2 of the large intestine can promote the adhesion between the biological tissue in the periphery on the mouth side A1 of the large intestine and the biological tissue on the intestinal wall on the anal side A2 of the large intestine. Therefore, it is possible to reduce risk factors of an anastomotic leakage after large intestine anastomosis.

According to this treatment method, an easy method is used in such a way that the sheet-like main body portion included in the adhesion promotion device is pinched between one joint target site and the other joint target site. The easy method can reduce the risk factors of the anastomotic leakage after a medical joining procedure (for example, anastomosis of a digestive tract).

In addition, in the adhesion promotion device 10 to be used, the rigidity of the main body portion 20 is improved by the reinforcement portion 30. Accordingly, it is possible to prevent the distortion or the misalignment from occurring in the adhesion promotion device 10 during the operation of the operator (when the adhesion promotion device 10 indwells the body). In addition, in a case where any force is applied after the indwelling, it is possible to prevent the distortion or the misalignment from occurring in the adhesion promotion device 10. Therefore, it is possible to reduce the risk factors of the anastomotic leakage after a surgical operation is performed.

This application is based on Japanese Patent Application No. 2018-182245 filed on September 27, 2018.

### Reference Signs List

- 10: adhesion promotion device
- 20: main body portion
- 21: front surface
- 23: rear surface
- 25: through-hole
- 30: reinforcement portion
- 31: first reinforcement portion
- 32: second reinforcement portion
- 35c: reinforcement portion layer
- 40: hole portion
- 50: virtual circle
- 51: center
- 52: line segment extending from center of virtual circle

## Claims

1. An adhesion promotion device (10) comprising:
a main body portion (20) formed of a biodegradable sheet having a plurality of through-holes (25) and is configured to promote adhesion of biological tissues; and
a reinforcement portion (30) disposed on a portion of the main body portion (20) and reinforcing the main body portion (20),
wherein the main body portion (20) further has a hole portion (40) formed to have a hole diameter larger than that of each of the plurality of through-holes (25),
wherein the reinforcement portion (30) includes a first reinforcement portion (30, 31) located on an outer edge side of the main body portion (20) and a second reinforcement portion (30, 32) located on an inner peripheral edge side of the hole portion (40),
wherein the second reinforcement portion (30, 32) has greater strength for reinforcing the biodegradable sheet forming the main body portion (20) than that of the first reinforcement portion (30, 31).

2. The adhesion promotion device (10) according to Claim 1,
wherein the reinforcement portion (30) intersects with a line segment (52) extending from a center (51) of a virtual circle (50) inscribed or circumscribed with the main body portion (20) at least at one location.

3. The adhesion promotion device (10) according to any one of Claims 1 to 2,
wherein an outer edge of the main body portion (20) includes a linear shape or an arc shape.

4. The adhesion promotion device (10) according to any one of Claims 1 to 3,
wherein the reinforcement portion (30) includes a linear shape or an arc shape.

## Patentansprüche

1. Vorrichtung (10) zur Vermittlung der Adhäsion, umfassend:
einen Hauptkörperabschnitt (20), der aus einer biologisch abbaubaren Folie gebildet ist, die eine Vielzahl von Durchgangslöchern (25) aufweist und so konfiguriert ist, dass sie die Adhäsion von biologischen Geweben vermittelt; und
einen Verstärkungsabschnitt (30), der an einem Abschnitt des Hauptkörperabschnitts (20) angeordnet ist und den Hauptkörperabschnitt (20) verstärkt,
wobei der Hauptkörperabschnitt (20) ferner einen Lochabschnitt (40) aufweist, der so ausgebildet ist, dass er einen Lochdurchmesser aufweist, der größer ist als derjenige von jedem der Vielzahl der Durchgangslöcher (25),
wobei der Verstärkungsabschnitt (30) einen ersten Verstärkungsabschnitt (30, 31), der an einer Außenkantenseite des Hauptkörperabschnitts (20) angeordnet ist, und einen zweiten Verstärkungsabschnitt (30, 32) umfasst, der an einer inneren Umfangskantenseite des Lochabschnitts (40) angeordnet ist,
wobei der zweite Verstärkungsabschnitt (30, 32) eine größere Festigkeit zur Verstärkung der den Hauptkörperabschnitt (20) bildenden biologisch abbaubaren Folie aufweist als derjenige des ersten Verstärkungsabschnitts (30, 31).

2. Vorrichtung (10) zur Vermittlung der Adhäsion nach Anspruch 1,
wobei der Verstärkungsabschnitt (30) ein Liniensegment (52) schneidet, das sich von einem Mittelpunkt (51) eines virtuellen Kreises (50) erstreckt, der dem Hauptkörperabschnitt (20) an mindestens einer Stelle eingeschrieben oder umschrieben ist.

3. Vorrichtung (10) zur Vermittlung der Adhäsion nach einem der Ansprüche 1 bis 2,
wobei eine Außenkante des Hauptkörperabschnitts (20) eine lineare Form oder eine Bogenform aufweist.

4. Vorrichtung (10) zur Vermittlung der Adhäsion nach einem der Ansprüche 1 bis 3,
wobei der Verstärkungsabschnitt (30) eine lineare Form oder eine Bogenform aufweist.

## Revendications

1. Dispositif de promotion d'adhérence (10) comprenant :
une partie de corps principal (20) formée d'une feuille biodégradable ayant une pluralité de trous traversants (25) et qui est configurée pour favoriser l'adhérence de tissus biologiques ; et
une partie de renfort (30) disposée sur une partie de la partie de corps principal (20) et renforçant la partie de corps principal (20),
dans lequel la partie de corps principal (20) a en outre une partie trou (40) formée pour avoir un diamètre de trou plus grand que celui de chacun de la pluralité de trous traversants (25),
dans lequel la partie de renfort (30) comporte une première partie de renfort (30, 31) située sur un côté de bord extérieur de la partie de corps principal (20) et une seconde partie de renfort (30, 32) située sur un côté de bord périphérique intérieur de la partie trou (40),
dans lequel la seconde partie de renfort (30, 32) a une force pour renforcer la feuille biodégradable formant la partie de corps principal (20) supérieure à celle de la première partie de renfort (30, 31).

2. Dispositif de promotion d'adhérence (10) selon la revendication 1,
dans lequel la partie de renfort (30) entrecoupe un segment de ligne (52) s'étendant à partir d'un centre (51) d'un cercle virtuel (50) inscrit dans la partie de corps principal (20), ou circonscrit à celle-ci, au niveau d'au moins un emplacement.

3. Dispositif de promotion d'adhérence (10) selon l'une quelconque des revendications 1 à 2,
dans lequel un bord extérieur de la partie de corps principal (20) comporte une forme linéaire ou une forme d'arc.

4. Dispositif de promotion d'adhérence (10) selon l'une quelconque des revendications 1 à 3,
dans lequel la partie de renfort (30) comporte une forme linéaire ou une forme d'arc.
